# EUROPEAN PATENT SPECIFICATION

(11) **EP 3 394 285 B1**
(45) Date of publication and mention of the grant of the patent: **22.06.2022**
(21) Application number: 16820240.6
(22) Date of filing: 20.12.2016
(51) Int. Cl.: C12Q 1/66

(54) **MICROBIAL CELL VIABILITY ASSAY FOR DETECTION OF OR DETERMINING SLURRY CONTAMINATION**
LEBENSFÄHIGKEITSPRÜFUNG MIKROBIELLER ZELLEN ZUR DETEKTION ODER BESTIMMUNG VON SCHLAMMKONTAMINATION
DOSAGE DE LA VIABILITÉ CELLULAIRE MICROBIENNE POUR DÉTECTER OU DÉTERMINER LA CONTAMINATION D'UNE PÂTE

(30) Priority: 22.12.2015 EP 15202170; 29.12.2015 US 201562272152 P
(43) Date of publication of application: 31.10.2018
(73) Proprietor: Omya International AG, 4665 Oftringen (CH)
(72) Inventor: GLAUBITZ, Joachim, 6264 Pfaffnau (CH); RUPP, Katharina, 4310 Rheinfelden (CH); BRÜLISAUER, Karin, 9000 St. Gallen (CH)
(74) Representative: Glas, Holger
(86) International application number: PCT/EP2016/081893
(87) International publication number: WO 2017/108768

(56) References cited:
- EP-A1- 0 816 512
- WO-A2-2005/033329
- "Quick Reference Guide QuenchGone21 TM Specialty Test Kit Product", LUMINULTRA , 6 April 2013 (2013-04-06), XP055269736, Retrieved from the Internet: URL:http://www.hach.com/quenchgone21-speci alty-test-kit-100-tests/product-downloads? id=15477405706 [retrieved on 2016-04-29]
- "Test Kit Instructions QuenchGone21 Speciality Test Kit", , 1 April 2013 (2013-04-01), XP055269735, Retrieved from the Internet: URL:http://www.hach.com/asset-get.download .jsa?id=15581727470 [retrieved on 2016-04-29]
- WEBSTER J J ET AL: "ATP in soil: A new extractant and extraction procedure", SOIL BIOLOGY AND BIOCHEMISTRY, PERGAMON, OXFORD, GB, vol. 16, no. 4, 1 January 1984 (1984-01-01), pages 335-342, XP023672477, ISSN: 0038-0717, DOI: 10.1016/0038-0717(84)90028-2 [retrieved on 1984-01-01]

## Description

The present invention relates to a method for detection of or determining an amount of microbial contamination in an aqueous preparation.

In practice, aqueous preparations and especially suspensions, dispersions or slurries of water-insoluble solids such as minerals, fillers or pigments are used extensively in the paper, paint, rubber and plastics industries as coatings, fillers, extenders and pigments for papermaking as well as aqueous lacquers and paints. For example, suspensions or slurries of calcium carbonate, talc or kaolin are used in the paper industry in large amounts as filler and/or as a component in the preparation of coated paper. Typical aqueous preparations of water-insoluble solids are characterized in that they comprise water, a water-insoluble solid compound and optionally further additives, such as dispersing agents, in the form of a suspension, a slurry or dispersion with a water-insoluble solid content of 0.1 to 99.0 wt.-% based on the total weight of the preparation. A typical aqueous preparation is a White Mineral Dispersion (WMD) having a solids content of 45.0 to 78.0 wt.-%. Water-soluble polymers and copolymers which may be used as e.g. dispersant and/or grinding aid in such preparation are, for example, described in US 5,278,248.

The aforementioned aqueous preparations are often subject to contamination by microorganisms such as fungi, yeasts, protozoa and/or aerobic and anaerobic bacteria resulting in changes in the preparation properties such as changes in viscosity and/or pH, discolorations or reductions in other quality parameters, which negatively affect their commercial value. Such aqueous preparations are thus usually tested by conventional methods such as plate count or dip slides for determining the microbial status of the preparation.

However, such tests typically take up to 48 hours to obtain a result regarding the microbial contamination and the question whether the preparation is to be treated with a biocide.

In the art, approaches for accelerating the detecting of or determining an amount of microbial contamination have been proposed. For example, US 2011/0076706 A1 describes analytical methods and related kits for detecting the presence of bacteria in a sample, determining the amount of bacteria in a sample, or determining the type of bacteria in a sample. In particular, it is described that a method for detecting the presence of or determining an amount of bacteria in a sample is provided, comprising: (a) determining a first amount of ATP (adenosine triphosphate) activity present in a sample; (b) contacting the sample of step (a) with a lytic agent at a concentration and for a time sufficient to lyse bacteria in said sample; and (c) determining a second amount of ATP activity present in the sample concurrent with or following step (b), thereby determining the presence of bacteria in the sample. The sample is preferably a biological sample selected from the group consisting of: blood, plasma, urine, platelets, bronchial lavage, saliva, wound fluid, tears, cerebrospinal fluid, amniotic fluid, and pleural fluid. WO 2000/046392 A1 relates to a method for quantitatively and/or qualitatively determining the microbial contamination of suspensions, emulsions or dispersions containing minerals and/or pigments and/or fillers and/or fibrous materials, characterised in that one or more organic substances which can be decomposed by micro-organisms is added to a sample of the suspensions, emulsions or dispersions, the sample is mixed, optionally incubated and then centrifuged in order to separate the micro-organisms from the minerals and/or pigments and/or fillers and/or fibrous materials. The number and/or size and/or type of micro-organisms in the supernatant aqueous phase is determined after one or more incubations.

However, such tests are developed for solutions which are free of particulate materials and, thus, the available test methods are insufficient for detecting of or determining a microbial contamination in aqueous preparations comprising a particulate material.

EP0816512 A1 discloses a bioluminescence test for detection of microorganisms in polymer- or pigment-containing dispersions and involves inter alia a step of diluting a sample of said dispersion to a weight ratio of 1 part polymer per 10 to 100 parts water.

The QuenchGone21^{™} Specialty Test Kit can be used for the determination of total microbiological concentration via ATP in chemical product testing, such as slurries, adhesives, paints, and other coatings, and involves a step of pre-dilution for highly turbid chemical products and a further step of dilution.

J. J. Webster at al. (Soil Biol. Biochem. 1984, 16, 335-342) report on a new extractant and extraction procedure for the determination of ATP in soil samples. The procedure involves a step of adding an extraction solution to the soil and a step of diluting the extract.

Thus, there is still a need for adequate methods for detection of or determining a microbial contamination in aqueous preparations comprising a particulate material and which result is obtained quickly.

Thus, it is an objective of the present invention to provide a method for detection of or determining a microbial contamination in aqueous preparations comprising a particulate material. In particular, it is thus an objective of the present invention to provide a method for detecting of or determining a microbial contamination in aqueous preparations comprising a particulate material which can be carried out quickly, preferably within a few minutes, for example, within 30 min. A further objective of the present invention is to provide a method for detecting of or determining a microbial contamination in aqueous preparations comprising a particulate material which can be carried without diluting the aqueous preparation beyond a factor of 2.

These and other objectives of the present invention can be solved by the method as described in the present invention and defined in the claims.

According to one aspect of the present application a method for detecting of or determining an amount of microbial contamination in an aqueous preparation is provided, the method comprising the steps of
a) providing an aqueous preparation comprising at least one particulate material,
b) providing at least one lytic agent,
c) contacting the aqueous preparation of step a) with the at least one lytic agent of step b) at a concentration and for a time sufficient to lyse a microbial contamination present in the aqueous preparation such that ATP (adenosine triphosphate) which is set free from the microbial contamination is dissolved in the aqueous preparation,
d) determining an amount of ATP content present in the aqueous preparation of step c), wherein the presence of microbial contamination is detected or the amount of microbial contamination is determined when the amount of ATP content is > 0,
wherein the aqueous preparation of step a) has a solids content from 10.0 to 82.0 wt.-%, based on the total weight of the aqueous preparation,
wherein the aqueous preparation of step a) is not diluted beyond a factor of 2 between method steps a) to d), and
wherein the aqueous preparation is an aqueous suspension

In accordance with the present invention, the term "aqueous" preparation refers to a system, wherein the liquid phase comprises, preferably consists of, water. However, said term does not exclude that the liquid phase of the aqueous preparation comprises minor amounts of at least one water-miscible organic solvent selected from the group comprising methanol, ethanol, acetone, acetonitrile, tetrahydrofuran and mixtures thereof. If the aqueous preparation comprises at least one water-miscible organic solvent, the liquid phase of the aqueous preparation comprises the at least one water-miscible organic solvent in an amount of from 0.1 to 40.0 wt.-% preferably from 0.1 to 30.0 wt.-%, more preferably from 0.1 to 20.0 wt.-% and most preferably from 0.1 to 10.0 wt.-%, based on the total weight of the liquid phase of the aqueous preparation. For example, the liquid phase of the aqueous preparation consists of water.

An aqueous "preparation" in the meaning of the present invention is a suspension or slurry comprising at least one particulate material and water and optionally further additives. Suspensions or slurries usually contain large amounts of particulate materials and are more viscous and generally of higher density than the liquid from which they are formed.

It is appreciated that the term "particulate material" in the meaning of the present invention refers to a water-insoluble material, preferably a material having a solubility in water of < 0.025 g/L at 25 °C and more preferably of < 0.015 g/L at 25 °C.

The term "lytic agent" in the meaning of the present invention refers to an agent suitable to lyse a microbial contamination such that ATP is set free from the microbial contamination present in the aqueous preparation.

Where the term "comprising" is used in the present description and claims, it does not exclude other elements. For the purposes of the present invention, the term "consisting of' is considered to be a preferred embodiment of the term "comprising of'. If hereinafter a group is defined to comprise at least a certain number of embodiments, this is also to be understood to disclose a group, which preferably consists only of these embodiments.

Where an indefinite or definite article is used when referring to a singular noun, e.g. "a", "an" or "the", this includes a plural of that noun unless something else is specifically stated.

Terms like "obtainable" or "definable" and "obtained" or "defined" are used interchangeably. This e.g. means that, unless the context clearly dictates otherwise, the term "obtained" does not mean to indicate that, e.g. an embodiment must be obtained by e.g. the sequence of steps following the term "obtained" even though such a limited understanding is always included by the terms "obtained" or "defined" as a preferred embodiment.

According to one embodiment of the present invention, the at least one particulate material is a white mineral having a whiteness of *R_{y}* of at least 65 % measured according to DIN 53163.

According to another embodiment of the present invention, the at least one particulate material of step a) is selected from the group comprising natural ground calcium carbonate, natural and/or synthetic precipitated calcium carbonate, dolomite, surface-modified calcium carbonate, hydromagnesite, hydroxyapatite, kaolin, talcum, barite, aluminium hydroxide, aluminium silicate, titanium dioxide and mixtures thereof, and preferably the at least one particulate material comprises natural ground calcium carbonate and/or synthetic precipitated calcium carbonate.

According to yet another embodiment of the present invention, the aqueous preparation of step a) has
(i) a pH value of from 2 to 12, preferably from 6 to 12 and more preferably from 7 to 10.5, and/or
(ii) a solids content from 20.0 to 80.0 wt.-%, based on the total weight of the aqueous preparation.

According to one embodiment of the present invention, the aqueous preparation of step a) is a paper making formulation, a paper coating formulation, fiber formulation, food formulation, pharmaceutical formulation, cosmetic formulation, plastic formulation and/or a paint formulation.

According to another embodiment of the present invention, the at least one particulate material is not separated from the aqueous phase of the aqueous preparation between method steps a) to d).

According to yet another embodiment of the present invention, the ATP is originated from a microbial contamination comprising gram-negative bacteria, gram-positive bacteria, fungi, moulds, yeasts, algae or mixtures thereof.

According to one embodiment of the present invention, the at least one lytic agent of step b) is selected from the group comprising trichloroacetic acid (TCA), perchloric acid (PCA), citric acid, cetyl trimethylammonium bromide (CTAB), dodecyl trimethyl ammonium bromide (DTAB), chlorhexidine (CHEX), ultrasonication, other nonionic, anionic and cationic detergents, bacterial specific lytic enzymes such as lysostaphin or lysozyme, proteinase such as proteinase K, antibiotics, alkylglucoside or alkylthioglucoside, betane detergents, quaternary ammonium salts, protamines, amines, and cationic, antibacterial, pore forming, membrane-active, and/or cell wall-active polymers, bacteriophage, surfactants such as triton X, and mixtures thereof.

According to another embodiment of the present invention, the at least one lytic agent of step b) yields sufficient lysis without inhibition by the presence of calcium and/or magnesium ions, preferably by the presence of bivalent cations and most preferably by the presence of the at least one particulate material of the aqueous preparation provided in step a).

According to yet another embodiment of the present invention, the at least one lytic agent of step b) is provided together with a sufficient amount of at least one chelating agent, preferably EDTA.

According to one embodiment of the present invention, the amount of ATP content is determined by using a luciferin/luciferase reagent assisted by luminescence detection.

According to another embodiment of the present invention, steps c) and d) are carried out simultaneously, or separately in the given order.

According to yet another embodiment of the present invention, steps c) and d) are carried out simultaneously by using a luciferin/luciferase reagent containing at least one lytic agent.

According to one embodiment of the present invention, the presence of microbial contamination is detected or the amount of microbial contamination is determined in step d) by monitoring the luminescence signal.

According to another embodiment of the present invention, the presence of microbial contamination is detected or the amount of microbial contamination is determined in step d) by using a luminometer.

According to yet another embodiment of the present invention, the amount of microbial contamination is determined in step d) by
(i) determining the amount of ATP content present in the aqueous preparation of step c),
(ii) treating the aqueous preparation of step a) such that the ATP content present in the aqueous preparation is essentially completely hydrolyzed and/or the microbial contamination present in the aqueous preparation is essentially completely killed, and processing the aqueous preparation through method steps a) to d), and
(iii) subtracting the amount of ATP content determined after step (ii) from the amount of ATP content determined in step (i).

As set out above, the inventive method for detecting of or determining an amount of microbial contamination in an aqueous preparation comprises the steps a), b), c) and d). In the following, it is referred to further details of the present invention and especially the foregoing steps of the inventive method for detecting of or determining an amount of microbial contamination in an aqueous preparation. Those skilled in the art will understand that many embodiments described herein can be combined or applied together.

### Characterization of step a): provision of an aqueous preparation

According to step a) of the method of the present invention, an aqueous preparation is provided.

It is appreciated that the aqueous preparation provided in step a) of the instant method can be any aqueous preparation that comprises at least one particulate material.

The aqueous preparation of step a) is an aqueous suspension.

The term "aqueous suspension" in the meaning of the present invention refers to a system comprising solvent and at least one inorganic particulate material and/or at least one organic material, wherein at least a part of the particles of the at least one inorganic particulate material is present as insoluble solids in the solvent.

In contrast thereto, the term "aqueous solution" refers to systems in which no discrete solid particles, i.e. particulate material particles, are observed in the solvent.

The aqueous preparation provided in step a) comprises at least one particulate material.

The term "at least one" particulate material in the meaning of the present invention means that the particulate material comprises, preferably consists of, one or more particulate materials.

In one embodiment of the present invention, the at least one particulate material comprises, preferably consists of, one particulate material. Alternatively, the at least one particulate material comprises, preferably consists of, two or more particulate materials. For example, the at least one particulate material comprises, preferably consists of, two or three particulate material.

Preferably, the at least one particulate material comprises, preferably consists of, one particulate material.

In one embodiment of the present invention, the at least one particulate material is at least one inorganic particulate material.

For example, the at least one particulate material, preferably the at least one inorganic particulate material, is a white mineral having a whiteness of *R_{y}* of at least 65 % measured according to DIN 53163. Preferably, the at least one particulate material, preferably the at least one inorganic particulate material, is a white mineral having a whiteness of *R_{y}* of at least 75 %, more preferably a whiteness of *R_{y}* of at least 80 % and most preferably a whiteness of *R_{y}* of at least 85 % measured according to DIN 53163.

It is appreciated that the at least one particulate material, for example the at least one inorganic particulate material, is preferably selected from the group comprising natural ground calcium carbonate, natural and/or synthetic precipitated calcium carbonate, dolomite, surface-modified calcium carbonate, hydromagnesite, hydroxyapatite, kaolin, talcum, barite, aluminium hydroxide, aluminium silicate, titanium dioxide and mixtures thereof.

In one embodiment of the present invention, the at least one particulate material, for example the at least one inorganic particulate material, comprises natural ground calcium carbonate and/or synthetic precipitated calcium carbonate.

"Ground calcium carbonate" (GCC) in the meaning of the present invention is a calcium carbonate obtained from natural sources, such as limestone, marble or chalk, and processed through a treatment such as grinding, screening and/or fractionizing by wet and/or dry, for example by a cyclone or classifier.

"Precipitated calcium carbonate" (PCC) in the meaning of the present invention is a synthesized material, generally obtained by precipitation following reaction of carbon dioxide and lime in an aqueous environment or by precipitation of a calcium and carbonate ion source in water.

A "surface-modified calcium carbonate" may feature surface-reacted GCC or PCC. A surface-reacted calcium carbonate may be prepared by providing a GCC or PCC in form of an aqueous suspension, and adding an H₃O⁺ ion donors to said suspension. Suitable H₃O⁺ ion donors are, for example, sulphuric acid, hydrochloric acid, phosphoric acid, citric acid, oxalic acid, or a mixture thereof. In a next step, the calcium carbonate is treated with gaseous carbon dioxide. If a strong H₃O⁺ ion donor such as sulphuric acid or hydrochloric acid is used for the H₃O⁺ ion donor treatment step, the carbon dioxide will form automatically *in situ.* Alternatively or additionally, the carbon dioxide can be supplied from an external source. Surface-reacted calcium carbonates are described, for example, in US 2012/0031576 A1, WO 2009/074492 A1, EP 2 264 109 A1, EP 2 070 991 A1, EP 2 264 108 A1, WO 00/39222 A1, WO 2004/083316 A1 or WO 2005/121257 A2.

"Hydromagnesite" in the meaning of the present invention is a hydrated magnesium carbonate containing mineral, having the chemical composition of Mg₅(CO₃)₄(OH)₂·4H₂O. It generally occurs associated with the weathering products of magnesium containing minerals such as serpentine or brucite.

"Dolomite" in the meaning of the present invention is a calcium carbonate containing mineral, namely a carbonic calcium-magnesium-mineral, having the chemical composition of CaMg(CO₃)₂ ("CaCO₃ · MgCO₃"). A dolomite mineral may contain at least 30.0 wt.-% MgCO₃, based on the total weight of dolomite, preferably more than 35.0 wt.-%, and more preferably more than 40.0 wt.-% MgCO₃.

The natural ground calcium carbonate and/or synthetic precipitated calcium carbonate and/or surface-modified calcium carbonate may additionally be surface treated. Thus, the natural ground calcium carbonate and/or synthetic precipitated calcium carbonate and/or surface-modified calcium carbonate may be treated with at least one hydrophobising agent to obtain a natural ground calcium carbonate and/or synthetic precipitated calcium carbonate comprising on at least a part of the accessible surface area a treatment layer comprising the hydrophobising agent.

The, hydrophobising agent can be selected from an aliphatic carboxylic acid having a total amount of carbon atoms from C4 to C24 and/or at least one mono-substituted succinic anhydride consisting of succinic anhydride mono-substituted with a group selected from a linear, branched, aliphatic and cyclic group having a total amount of carbon atoms from C2 to C30 in the substituent and/or a phosphoric acid ester blend of one or more phosphoric acid mono-ester and one or more phosphoric di-ester.

Processes for treating the natural ground calcium carbonate and/or synthetic precipitated calcium carbonate and/or surface-modified calcium carbonate with at least one mono-substituted succinic anhydride and/or with at least one phosphoric acid ester blend and suitable compounds for surface treatment are described in EP 2 722 368 A1 and EP 2 770 017 A1.

Suitable aliphatic carboxylic acids for treating the natural ground calcium carbonate and/or synthetic precipitated calcium carbonate are for example aliphatic linear or branched carboxylic acids having between 4 and 24 carbon atoms.

The aliphatic linear or branched carboxylic acid in the meaning of the present invention may be selected from one or more straight chain, branched chain, saturated, unsaturated and/or alicyclic carboxylic acids. Preferably, the aliphatic linear or branched carboxylic acid is a monocarboxylic acid, i.e. the aliphatic linear or branched carboxylic acid is characterized in that a single carboxyl group is present. Said carboxyl group is placed at the end of the carbon skeleton.

In one embodiment of the present invention, the aliphatic linear or branched carboxylic acid is selected from saturated unbranched carboxylic acids, that is to say the aliphatic linear or branched carboxylic acid is preferably selected from the group of carboxylic acids consisting of butanoic acid, pentanoic acid, hexanoic acid, heptanoic acid, octanoic acid, nonanoic acid, decanoic acid, undecanoic acid, lauric acid, tridecanoic acid, myristic acid, pentadecanoic acid, palmitic acid, heptadecanoic acid, stearic acid, nonadecanoic acid, arachidic acid, heneicosanoic acid, behenic acid, tricosanoic acid, lignoceric acid and mixtures thereof.

In another embodiment of the present invention, the aliphatic linear or branched carboxylic acid is selected from the group consisting of octanoic acid, decanoic acid, lauric acid, myristic acid, palmitic acid, stearic acid, arachidic acid and mixtures thereof. Preferably, the aliphatic linear or branched carboxylic acid is selected from the group consisting of myristic acid, palmitic acid, stearic acid and mixtures thereof.

For example, the aliphatic linear or branched carboxylic acid is stearic acid.

The at least one particulate material, preferably the at least one inorganic particulate material, of the aqueous preparation provided in step a) may have a particle size distribution as conventionally employed for the material(s) involved in the type of product to be tested. In general, 90 % of the particles will have an esd (equivalent spherical diameter as measured by the well known technique of sedimentation using Sedigraph 5120 series, Micromeritics) of less than 6 micrometres (µm). Coarse particulate materials may have a particle esd generally (i.e. at least 90 wt.-%) in the range of 1 to 5 µm. Fine particulate materials may have a particle esd generally less than 2 µm, e.g. 50.0 to 99.0 wt.-% less than 2 µm and preferably 60.0 to 90.0 wt.-% less than 2 µm. It is preferred that the at least one particulate material in the aqueous preparation has a weight median particle size *d*₅₀ value of from 0.1 to 5 µm, preferably from 0.2 to 2 µm and most preferably from 0.35 to 1 µm, for example 0.7 µm as measured using a Sedigraph^{™} 5120 of Micromeritics Instrument Corporation.

For keeping such inorganic particulate materials, preferably inorganic particulate materials, dispersed in an aqueous preparation and thus ensuring that the viscosity of the preparation remains substantially the same over time, additives such as dispersing agents can be present in the aqueous preparation of step a). A suitable dispersing agent according to the present invention is preferably a homo- or copolymer made of monomers and/or co-monomers selected from the group consisting of acrylic acid, methacrylic acid, itaconic acid, crotonic acid, fumaric acid, maleic anhydride acid, isocrotonic acid, aconitic acid (cis or trans), mesaconic acid, sinapinic acid, undecylenic acid, angelic acid, canellic acid, hydroxyacrylic acid, acrolein, acrylamide, acrylonitrile, dimethylaminoethyl methacrylate, vinylpyrrolidone, styrene, the esters of acrylic and methacrylic acids and mixtures thereof, wherein salts of poly(acrylic acid) and/or poly (methacrylic acid) are preferred as dispersing agent.

Additionally or alternatively, the aqueous preparation of step a) comprises at least one organic particulate material. For example, the at least one organic particulate material is selected from the group comprising, glycols, carbohydrates such as CMC or starch, cellulose and cellulose based pulp, glycerol and mixtures thereof.

In one embodiment of the present invention, the aqueous preparation of step a) comprises at least one inorganic particulate material and at least one organic particulate material. Alternatively, the aqueous preparation of step a) comprises at least one inorganic particulate material or at least one organic particulate material.

Preferably, the aqueous preparation of step a) comprises at least one particulate material being at least one inorganic particulate material. More preferably, the at least one particulate material is at least one inorganic particulate material being selected from the group comprising natural ground calcium carbonate, natural and/or synthetic precipitated calcium carbonate, dolomite, surface-modified calcium carbonate, hydromagnesite, hydroxyapatite, kaolin, talcum, barite, aluminium hydroxide, aluminium silicate, titanium dioxide and mixtures thereof, and most preferably the at least one particulate material comprises natural ground calcium carbonate and/or synthetic precipitated calcium carbonate.

The solids content of the aqueous preparation is from 10.0 to 82.0 wt.-%, and more preferably from 20.0 to 80.0 wt.-%, based on the dry weight of CaCO₃.

The pH of the aqueous preparation provided in step a) can vary in a broad range and is preferably in a pH range typically observed for such aqueous preparations. It is thus appreciated that the aqueous preparation of step a) preferably has a pH value of from 2 to 12. For example, the aqueous preparation of step a) has a pH value of from 6 to 12 and more preferably from 7 to 10.5. Unless indicated otherwise, the pH is measured in accordance with the method set out in the example section.

Typically, the aqueous preparation provided in step a) has a viscosity being preferably in the range from 50 to 2 000 mPa·s and preferably from 80 to 800 mPa·s, as measured with a Brookfield DV-II Viscometer at a speed of 100 rpm and equipped with a LV-3 spindle.

The aqueous preparations according to the invention can be produced by methods known in the art, by for example, dispersing, suspending or slurring water-insoluble solids, preferably inorganic particulate materials with, if appropriate, addition of a dispersing agent and, if appropriate, further additives in water.

The aqueous preparation provided in step a) can be any aqueous preparation of which the microbial status is to be determined provided that the aqueous preparation comprises at least one particulate material.

For example, the aqueous preparation of step a) is a paper making formulation, a paper coating formulation, fibre formulation, food formulation, pharmaceutical formulation, cosmetic formulation, plastic formulation and/or a paint formulation.

### Characterisation of step b): provision of at least one lytic agent

According to step b) of the method of the present invention, at least one lytic agent is provided.

The term "at least one" lytic agent in the meaning of the present invention means that the lytic agent comprises, preferably consists of, one or more lytic agents.

In one embodiment of the present invention, the at least one lytic agent comprises, preferably consists of, one lytic agent. Alternatively, the at least one lytic agent comprises, preferably consists of, two or more lytic agents. For example, the at least one lytic agent comprises, preferably consists of, two or three lytic agents.

Preferably, the at least one lytic agent comprises, preferably consists of, one lytic agent.

It is appreciated that the at least one lytic agent may by any agent that is suitable for altering cell membranes or cell wall permeability or disrupting the integrity of the membrane or cell wall of a microbial contamination such that lyses or the formation of pores is caused such that ATP is set free from the microbial contamination which may be present in the aqueous preparation.

Preferred lytic agents according to the present invention include lytic agents selected from the group comprising trichloroacetic acid (TCA), perchloric acid (PCA), citric acid, cetyl trimethylammonium bromide (CTAB), dodecyl trimethyl ammonium bromide (DTAB), chlorhexidine (CHEX), ultrasonication, other nonionic, anionic and cationic detergents, bacterial specific lytic enzymes such as lysostaphin or lysozyme, proteinase such as proteinase K, antibiotics, alkylglucoside or alkylthioglucoside, betane detergents, quaternary ammonium salts, protamines, amines, and cationic, antibacterial, pore forming, membrane-active, and/or cell wall-active polymers, bacteriophage, surfactants such as triton X, and mixtures thereof.

In one embodiment of the present invention, the at least one lytic agent of step b) is selected from the group comprising trichloroacetic acid (TCA), perchloric acid (PCA), citric acid, cetyl trimethylammonium bromide (CTAB), dodecyl trimethyl ammonium bromide (DTAB), chlorhexidine (CHEX), ultrasonication, other nonionic, anionic and cationic detergents, surfactants such as triton X, and mixtures thereof. For example, the at least one lytic agent of step b) is selected from the group comprising trichloroacetic acid (TCA), cetyl trimethylammonium bromide (CTAB), surfactants such as triton X, and mixtures thereof.

It is appreciated that the at least one lytic agent of step b) yields sufficient lysis without inhibition by the presence of calcium and/or magnesium ions.

Preferably, the at least one lytic agent of step b) yields sufficient lysis without inhibition by the presence of bivalent cations. The term "bivalent cation" in the meaning of the present invention refers to a cation having a valency of two, e.g. a metal cation having two valencies. For example, the bivalent cation can be selected from the group consisting of calcium ions, magnesium ions, strontium ions and mixtures thereof. The bivalent cation may be provided as salts or halides such as sulphate, sulphonate, gluconate, carbonate, chloride or bromide.

Most preferably, the at least one lytic agent of step b) yields sufficient lysis without inhibition by the presence of the at least one particulate material of the aqueous preparation provided in step a). The at least one particulate material is preferably selected from the group comprising natural ground calcium carbonate, natural and/or synthetic precipitated calcium carbonate, dolomite, surface-modified calcium carbonate, hydromagnesite, hydroxyapatite, kaolin, talcum, barite, aluminium hydroxide, aluminium silicate, titanium dioxide and mixtures thereof, and most preferably the at least one particulate material comprises natural ground calcium carbonate and/or synthetic precipitated calcium carbonate.

In one embodiment of the present invention, the at least one lytic agent of step b) is provided together with a sufficient amount of at least one chelating agent. It is appreciated that the simultaneous provision of the at least one lytic agent of step b) and the at least one chelating agent is advantageous as the chelating agent may scavenger free cations such as bivalent cations and thus prevents their action of inhibition.

The at least one chelating agent includes, without limitation, salts of ethylenediamine tetraacetic acid (EDTA), ethylene glycol tetraacetic acid (EGTA), and 1,2-Cyclohexanedinitrilotetraacetic acid (CDTA), nitriloacetic acid (NTA), citric acid, sodium gluconate, gluconic acid, lignosulfonates, and mixtures thereof. Preferably, the at least one chelating agent is selected from the group consisting of EDTA and EGTA, due to their general availability and relatively low cost. Most preferably, the at least one chelating agent is EDTA.

The at least one chelating agent is preferably provided in a sufficient concentration. The sufficient concentration may be empirically determined on the basis of providing levels sufficient to provide enough free ATP from the cell lysis as well as levels which do not inhibit the ATP-determination.

Preferably, the at least one chelating agent is provided in an amount which corresponds to at least about 10% of the at least one lytic agent amount, preferably about 20%, 30%, 40%, 50%, or 60% of the at least one lytic agent amount.

One of skill in the art will recognize, however, that different chelating agents may have different chelating capacities depending on the pH. Thus, the outer parameters of the present invention include a degree of variability in the amount of the at least one chelating agent provided that the chelating capacity is comparable to that of EDTA (ethylenediamine tetraacetic acid) under otherwise identical luciferase assay reaction conditions (at e.g., pH 7.0-10.5 etc.). In other words, the amount of the at least one chelating agent may be adjusted to provide a chelating capacity comparable or exceeding the chelating capacity of EDTA under otherwise identical conditions (all other reagents and reagent concentrations same, except for the at least one chelating agent).

### Characterization of step c): contacting the aqueous preparation with the at least one lytic agent

According to step c) of the method of the present invention, the aqueous preparation of step a) is contacted with the at least one lytic agent of step b). It is one requirement of the instant invention that the aqueous preparation of step a) is contacted with the at least one lytic agent of step b) at a concentration and for a time sufficient to lyse a microbial contamination present in the aqueous preparation such that ATP which is set free from the microbial contamination is dissolved in the aqueous preparation.

It is thus appreciated that the aqueous preparation of step a) may comprise a microbial contamination which is to be detected or which amount is to be determined in the aqueous preparation. The aqueous preparation of step a) may comprise a microbial contamination typically found in the aqueous preparations to be analysed, for example paper making formulations, paper coating formulations, fibre formulations, food formulations, pharmaceutical formulations, cosmetic formulations, plastic formulations and/or a paint formulations.

Preferably, the microbial contamination comprises gram-negative bacteria, gram-positive bacteria, fungi, moulds, yeasts or mixtures thereof.

In one embodiment of the present invention, the microbial contamination comprises, preferably consists of, gram-negative bacteria, gram-positive bacteria, fungi, moulds, algae or yeasts. Alternatively, the microbial contamination comprises, preferably consists of, two or more microbial contaminations independently selected from gram-negative bacteria, gram-positive bacteria, fungi, moulds, algae and yeasts. For example, the microbial contamination comprises, preferably consists of, two or three microbial contaminations independently selected from gram-negative bacteria, gram-positive bacteria, fungi, moulds, algae and yeasts. Preferably, the microbial contamination comprises, preferably consists of, two or more microbial contaminations independently selected from gram-negative bacteria, gram-positive bacteria, fungi, moulds, algae and yeasts.

It is appreciated that gram-positive and gram-negative bacteria are well known in the art and are e.g. described in Biology of Microorganisms, "Brock", Madigan MT, Martinko JM, Parker J, 1997, 8th Edition. In particular, such bacteria represent evolutionary very distantly related classes of bacteria each comprising of many bacterial families. Gram negative bacteria are characterized by two membranes (outer and inner membrane) while gram positive bacteria contain only one membrane. Usually, the former contains a high amount of lipopolysaccharide and a thin single-layer of peptidoglycan, while the latter has virtually no lipopolysaccharide, a multi-layered thick peptidocglycan and the coat contains teichoic acids. For these differences the Gram positive and Gram negative bacteria react differently on environmental influences. Methods for discriminating gram-positive and gram-negative bacteria include species identification by DNA sequencing techniques or biochemical characterizations. Alternatively, the number of membranes can be determined directly by thin section transmission electron microscopy.

For example, the gram-negative bacteria and gram-positive bacteria can be selected from the group comprising *Methylobacterium sp., Salmonella sp., Escherichia sp.* such as *Escherichia coli, Shigella sp., Enterobacter sp., Pseudomonas sp.* such as *Pseudomonas mendocina, Bdellovibrio sp., Agrobacterium sp., Alcaligenes sp., Flavobacterium sp., Rhizobium sp., Sphingobacterium sp., Aeromonas sp., Chromobacterium sp., Vibrio sp., Hyphomicrobium sp., Leptothrix sp., Micrococcus sp., Staphylococcus sp.* such as *Staphylococcus aureus, Agromyces sp., Acidovorax sp.,* and mixtures thereof.

For example, the gram-negative bacteria and gram-positive bacteria are selected from *Escherichia sp.* such as *Escherichia coli, Staphylococcus sp.* such as *Staphylococcus aureus,* and mixtures thereof.

Additionally or alternatively, the yeast can be selected from the group comprising *Saccharomycotina, Taphrinomycotina, Schizosaccharomycetes, Basidiomycota, Agaricomycotina, Tremellomycetes, Pucciniomycotina, Microbotryomycetes, Candida sp.* such as *Candida albicans, Candida tropicalis, Candida stellatoidea, Candida glabrata, Candida krusei, Candida guilliermondii, Candida viswanathii, Candida lusitaniae and mixtures thereof, Yarrowia sp.* such as *Yarrowia lipolytica, Cryptococcus sp.* such as *Cryptococcus gattii* and *Cryptococcus neofarmans, Zygosaccharomyces sp., Rhodotorula sp.* such as *Rhodotorula mucilaginosa,* and mixtures thereof.

Additionally or alternatively, the mould can be selected from the group comprising *Acremonium sp., Alternaria sp., Aspergillus sp., Cladosporium sp., Fusarium sp., Mucor sp., Penicillium sp., Rhizopus sp., Stachybotrys sp., Trichoderma sp., Dematiaceae sp., Phoma sp., Eurotium sp., Scopulariopsis sp., Aureobasidium sp., Monilia sp., Botrytis sp., Stemphylium sp., Chaetomium sp., Mycelia sp., Neurospora sp., Ulocladium sp., Paecilomyces sp., Wallemia sp., Curvularia sp.,* and mixtures thereof.

It is appreciated that the at least one lytic agent is added to the aqueous preparation at a concentration and for a time sufficient to lyse the microbial contamination present in the aqueous preparation such that ATP which is set free from the microbial contamination is dissolved in the aqueous preparation.

In general, the amount of the at least one lytic agent sufficient to lyse the microbial contamination present in the aqueous preparation may vary depending on the microbial contamination, if present, and the at least one lytic agent used. The amount of the at least one lytic agent may be empirically determined using common methods known to the skilled person or described in the present application.

In one embodiment of the present invention, the at least one lytic agent is added to the aqueous preparation at a concentration in the range from 0.01 wt.-% to 5.0 wt.-%, based on the total weight of the aqueous preparation. For example, if a surfactant such as triton X is used as the at least one lytic agent, the at least one lytic agent is added to the aqueous preparation at a concentration in the range from 0.25 wt.-% to 1.0 wt.-%, based on the total weight of the aqueous preparation. If cetyl trimethylammonium bromide (CTAB) is used as the at least one lytic agent, the at least one lytic agent is added to the aqueous preparation at a concentration in the range from 0.02 wt.-% to 0.08 wt.-%, based on the total weight of the aqueous preparation.

In general, the aqueous preparation of step a) and the at least one lytic agent of step b) can be brought into contact by any conventional means known to the skilled person.

It is appreciated that contacting step c) is preferably carried out by adding the at least one lytic agent of step b) to the aqueous preparation of step a).

In one embodiment of the present invention, the step of contacting the aqueous preparation of step a) with the at least one lytic agent of step b) is carried out in that the at least one lytic agent is added to the aqueous preparation under mixing. A sufficient mixing may be achieved by shaking the aqueous preparation or by agitation, which may provide a more thorough mixing. In one embodiment of the present invention, contacting step c) is carried out under agitation to ensure a thorough mixing of the aqueous preparation and the at least one lytic agent. Such agitation can be carried out continuously or discontinuously.

It is one requirement of the present invention that contacting step c) is carried out for a time sufficient to lyse a microbial contamination present in the aqueous preparation such that ATP which is set free from the microbial contamination is dissolved in the aqueous preparation. It is thus appreciated that the contacting is carried out for a time such that no further increase of the ATP amount is detected in the aqueous preparation. The contacting time may be empirically determined using common methods known to the skilled person or described in the present application.

For example, a sufficient time for contacting the aqueous preparation with the at least one lytic agent in step c) is in the range from 3 to 30 min, preferably in the range from 3 to 20 min, more preferably in the range from 3 to 10 min, and most preferably about 5 min. The contacting typically starts when the at least one lytic agent is thoroughly mixed into the aqueous preparation.

It is appreciated that contacting step c) can be repeated one or more times.

The aqueous preparation obtained in step c) preferably has solids content corresponding to the solids content of the aqueous preparation provided in step a). It is thus appreciated that the aqueous preparation obtained in step c) preferably has solids content from 10.0 to 82.0 wt.-%, and preferably from 20.0 to 80.0 wt.-%, based on the total weight of the aqueous preparation obtained in step c).

Additionally or alternatively, the pH of the aqueous preparation obtained in step c) preferably corresponds to the pH of the aqueous preparation provided in step a). It is thus appreciated that the aqueous preparation obtained in step c) preferably has a pH value of from 2 to 12. For example, the aqueous preparation obtained in step c) has a pH value of from 6 to 12 and more preferably from 7 to 10.5.

Typically, the aqueous preparations obtained in step c) has a viscosity being preferably in the range between 50 and 2 000 mPa·s, and preferably 80 and 800 mPa·s, as measured with a Brookfield DV-II Viscometer at a speed of 100 rpm and equipped with a LV-3 spindle.

### Characterization of step d): determining an amount of ATP content

According to step c) of the method of the present invention, an amount of ATP content is determined. It is one requirement of the present invention that the presence of microbial contamination is detected or the amount of microbial contamination is determined when the amount of ATP content is > 0.

It is appreciated that the detection or the determination of the microbial contamination can be carried out by every means known to the skilled person. For example, an ATP-dependent luminescence-producing enzyme can be used for determining the amount of ATP content. Preferably, the amount of ATP content is determined by using a luciferin/luciferase reagent.

Luciferin/luciferase reagents are well known to the skilled person. In general, luciferases are defined by their ability to produce luminescence. More precisely, luciferases are known to catalyse the oxidation of a substrate, luciferin, thereby producing oxyluciferin and photons. Luciferases, whose catalytic products include light, offer sensitivity, a detectable product, and thus enable the determination of an amount of ATP content present in the aqueous preparation.

For example, beetle luciferases such as that of the common firefly (family Lampyridae) can be used in the present invention. Beetle luciferases are often referred to as firefly luciferases; however, firefly luciferases are actually a subgroup of the beetle luciferase class. Beetle luciferases may be purified from the lanterns of the beetles themselves or from protein expression systems well known in the art.

It is appreciated that all luciferases, luciferase variants, luciferase fragments and variant luciferase fragments that catalyse an ATP-dependent reaction and generate luminescence can be used for the present invention. It is appreciated that the luciferase, luciferase variant, luciferase fragment and variant luciferase fragment are preferably selected such that it is stable under the pH conditions of the aqueous preparation provided in step a), more preferably at a pH from 2 to 12, even more preferably from 6 to 12 and most preferably from 7 to 10.5. In particular, beetle luciferases, particularly firefly luciferase from the North American firefly Photinus pyralis, can be used. Alternatively, the P. pyralis luciferase (LucPpy) consisting of approximately 550 amino acids of Mr 61 kDa as calculated by the protein encoded by the nucleotide sequence of the gene can be used. Other firefly luciferases in accordance with the present invention include Photuris pennsylvanica firefly luciferase (LucPpe2; 545 amino acid residues; GenBank 2190534, as well as various mutant luciferases disclosed in U.S. 2003/0104507, which are derived from LucPpe2 (e.g., LucPpe2 m78 (also known as 78-0B10); LucPpe2 m90 (also known as 90-1B5); LucPpe2 m133 (also known as 133-1B2); LucPpe2m146 (also known as 146-1H2); and various commercially available luciferases, such as UltraGlo(TM) Luciferase (Promega). Methods for making LucPpe2m78, LucPpe2m90, LucPpe2m133, and LucPpe2m146 are disclosed in U.S. 2003/0104507.

Preferably, the luciferase is provided in isolated and/or purified form. The purity can be determined by any means known to the skilled person. For example, the purity can be determined by using SDS-PAGE analysis under non-reducing or reducing conditions using Coomassie blue or silver stain. Luciferases may be isolated from native luciferase-producing sources or from a recombinant cell expressing an exogenous luciferase-encoded polynucleotide. Methods for producing luciferases are well known to those of skill in the art.

The naturally-occurring substrate for beetle luciferases is firefly luciferin. Luciferin is a polytherocyclic organic acid, D-(-)-2-(6'-hydroxy-2'-benzothiazolyl)-Δ²- thiazolin-4-carboxylic acid (luciferin). Luciferin may be isolated from natural sources such as from fireflies or synthesized. Synthetic luciferin can have the same structure as the naturally occurring luciferin or can be a variant or derivative, as long as it functions analogously. Exemplary luciferin derivatives for use in the present invention include, but are not limited to, 6-deoxyaminoluciferin, D-luciferin methyl ester, D-luciferyl-L-phenylalanine, D-luciferyl-L-N α-arginine, D-luciferin-O-sulphate and D-luciferin-O-phosphate, esters of luciferases that are hydrolysed or acted upon by esterases to luciferin by components in a sample. Other examples of useful luciferin analogues include naphthyl- and quinolylluciferin, which emit light in the green and red light spectra respectively.

The luciferase-catalysed reaction produces a luminescent signal from the luciferaseluciferin reaction.

It is thus preferred that the presence of microbial contamination is detected or the amount of microbial contamination is determined in step d) by monitoring the luminescence signal. Such monitoring can be carried out by each means known to the skilled person. For example, the presence of microbial contamination is detected or the amount of microbial contamination is determined in step d) by using a luminometer. Luminometers are well known to the skilled person and are, e.g., available from Promega Corporation, USA.

In one embodiment of the present invention, step d) is carried out during or after contacting step c).

For example, step d) is carried out during contacting step c). Alternatively, step d) is carried out after contacting step c).

Accordingly, steps c) and d) are carried out simultaneously, or separately in the given order. For example, steps c) and d) are carried out separately in the given order, i.e. step d) is carried out after step c). Alternatively, steps c) and d) are carried out simultaneously.

Preferably, steps c) and d) are carried out simultaneously by using a luciferin/luciferase reagent containing at least one lytic agent.

In one embodiment, the luciferin/luciferase reagent containing at least one lytic agent used is the BacTiter-Glo^{™} Reagent (Promega Corp., Madison, Wis., USA).

In one embodiment of the present invention, the amount of microbial contamination is determined in step d) by
(i) determining the amount of ATP content present in the aqueous preparation of step c),
(ii) treating the aqueous preparation of step a) such that the ATP content present in the aqueous preparation is essentially completely hydrolysed and/or the microbial contamination present in the aqueous preparation is essentially completely killed, and processing the aqueous preparation through method steps a) to d), and
(iii) subtracting the amount of ATP content determined after step (ii) from the amount of ATP content determined in step (i).

It is appreciated that treating step (ii) can be carried by any means known to the skilled person which is suitable for essentially completely hydrolysing the ATP content present in the aqueous preparation and/or for essentially completely killing the microbial contamination present in the aqueous preparation. Hydrolysing of the ATP content and/or killing of the microbial contamination can be carried out by methods well known in the art. For example, such hydrolysing and/or killing can be achieved by autoclaving or sterilizing the aqueous preparation.

Additionally or alternatively, the amount of microbial contamination is determined in step d) by
(i) determining the amount of ATP content present in the aqueous preparation of step d) after its processing through to step d) by omitting step c),
(ii) determining the amount of ATP content present in the aqueous preparation of step a) after its processing through to step d), and
(iii) subtracting the amount of ATP content determined in step (i) from the amount of ATP content determined in step (ii).

It is one requirement of the present invention that the aqueous preparation provided in step a) comprises at least one particulate material. It is preferred that also the aqueous preparation of step d) comprises at least one particulate material. Preferably the at least one particulate material of the aqueous preparation of step d) is the same as of the aqueous preparation provided in step a).

Thus, it is preferred that the at least one particulate material is not separated from the aqueous phase of the aqueous preparation between method steps a) to d).

It is further appreciated that the aqueous preparation of step a) is not diluted beyond a specific factor.

The aqueous preparation of step a) is not diluted beyond a factor of 2.

In one embodiment of the present invention, the at least one particulate material is not separated from the aqueous phase of the aqueous preparation between method steps a) to d) and the aqueous preparation of step a) is not diluted beyond a factor of 2.

The aqueous preparation obtained in step d) thus preferably has solids content corresponding, i.e. is equal or as close as possible, to the solids content of the aqueous preparation provided in step a) and/or to the solids content of the aqueous preparation obtained in step c). It is thus appreciated that the aqueous preparation obtained in step d) preferably has solids content from 10.0 to 82.0 wt.-%, and preferably from 20.0 to 80.0 wt.-%, based on the total weight of the aqueous preparation obtained in step d).

Additionally or alternatively, the pH of the aqueous preparation obtained in step d) preferably corresponds, i.e. is equal or as close as possible, to the pH of the aqueous preparation provided in step a) and/or to the pH of the aqueous preparation obtained in step c). It is thus appreciated that the aqueous preparation obtained in step d) preferably has a pH value of from 2 to 12. For example, the aqueous preparation obtained in step d) has a pH value of from 6 to 12 and more preferably from 7 to 10.5.

Typically, the aqueous preparations obtained in step d) has a viscosity being preferably in the range between 50 and 2 000 mPa·s, and preferably 80 and 800 mPa·s, as measured with a Brookfield DV-II Viscometer at a speed of 100 rpm and equipped with a LV-3 spindle.

The inventive method thus provides a number of improved properties. First of all, the inventive method is suitable for detecting of or determining a microbial contamination in aqueous preparations comprising a particulate material with certain viscosity. Furthermore, the inventive method can be carried out quickly, preferably within a few minutes, for example, within 30 min with a minimum of working steps (e.g. no separation of the particulate material, minimum dilutions). In addition thereto, the inventive method can be carried out without diluting the aqueous preparation beyond a factor of 2. The lysis reaction is robust against inhibition of the aqueous preparation and its particulate material (e.g. it is containing bivalent cations). The detection reaction (and or the enzyme for it) is stable within the pH range of the aqueous preparation and its particulate material (e.g. pH 7-10.5). The inventive method thus allows for determining the microbial status of an aqueous preparation very quickly such that direct action by treating the aqueous preparation with a biocide is possible, if necessary.

The following examples may additionally illustrate the invention, but are not meant to restrict the invention to the exemplified embodiments.

### Examples

### 1. Materials

Calcium carbonate slurries A, B and C have been used. The characteristics of these calcium carbonate slurries are outlined in the following table 1:

**Table 1: Characteristics of the calcium carbonate slurries**

| | **Slurry A** | **Slurry B** | **Slurry C** |
|---|---|---|---|
| **Residue > 45 µm** | 10 ppm | - | 50 ppm |
| **Residue > 75 µm** | - | 10 ppm | - |
| **Top cut (*d*₉₈)** | 4 µm | 30 µm | - |
| **Median particle size by weight at constant density (Sedigraph), or by volume (HELOS) (*d*₅₀)** | 0.7 µm | 5.7 µm | Sedigraph 5120: 1.6-2.2 µm Sympatec HELOS: 2.2-3.0 µm |
| **Particle fraction by weight at constant density (Sedigraph), or by volume (HELOS) < 2 µm** | 90% | 24% | Sedigraph 5120: 50-65% Sympatec HELOS: 30-45% |
| **Tappi-whiteness [%]** | 94 | - | 95-97.5 |
| **Whiteness CIE *L**, *a**, *b**** | - | 98/0.3/0.9 | - |
| **Ry (C/2°)[%]** | - | 96% | - |
| **Solid content [wt.-%]** | 76,50 | 70 | 48-53 |
| **Density[kg m⁻³]** | 1 929 | 1 790 | - |
| **pH** | 10 | 9.2 | 8-10 |
| **Viscosity [mPa*s]** | 350 | 1 000 | 600 |

### 2. Measurement methods

The following measurement methods are used to evaluate the parameters given in the examples and claims.

### Slurry pH measurement

The pH of a slurry (or suspension) is measured at 20 to 25°C using a Mettler Toledo Seven Easy pH meter and a Mettler Toledo InLab^{®} InLab413 SG pH electrode. A three point calibration (according to the segment method) of the instrument is first made using commercially available buffer solutions (e.g. Hamilton Duracal buffer solutions) having pH values of 4.01, 7.0 and 9.2 at 21.7 °C (from Aldrich). The reported pH values are the endpoint values detected by the instrument (the endpoint is when the measured signal differs by less than 0.1 mV from the average over the last 6 seconds).

### The pH range of the calcium carbonate slurries tested is between

- 8.5-10 (100 g/L [20°C]) for calcium carbonate slurry A
- 8.5-10.7 (100 g/L [20°C]) for calcium carbonate slurry B
- 8.5-11.5 (100 g/L [20°C]) for calcium carbonate slurry C

### Solids content of an aqueous slurry

The slurry solids content (also known as "dry weight") is determined using a Moisture Analyser HR73 commercially available from Mettler-Toledo, Switzerland, with the following settings: temperature of 120°C, automatic switch off 3, standard drying of 5-20 g of slurry.

### Particle size distribution (mass % particles with a diameter < X) and weight median diameter (d₅₀) of a particulate calcium carbonate-containing material

Weight median grain diameter and grain diameter mass distribution of a particulate calcium carbonate-containing material were determined via the sedimentation method, i.e. an analysis of sedimentation behaviour in a gravimetric field. The measurement was made with a Sedigraph TM 5120. Alternatively, the measurement can be made via laser diffraction, i.e. the particle size is determined in respect to particle volume by measuring the intensity of light scattered as a laser beam passes through a dispersed particulate sample assuming the particles approximate to a sphere. The measurement was made with a HELOS particle-size-analyser of Sympatec, Germany, and may be considered equivalent to weight distribution assuming a constant density throughout the particle size distribution.

The method and the instruments are known to the skilled person and are commonly used to determine grain size of fillers and pigments. The measurement is carried out in an aqueous solution of 0.1 wt.-% Na₄P₂O₇. The samples are dispersed using a high speed stirrer and supersonics.

### Density

Density measurements were made in accordance with DIN EN 323.

### Viscosity

For the purpose of the present invention, the term "viscosity" or "Brookfield viscosity" refers to Brookfield viscosity. The Brookfield viscosity is for this purpose measured by a Brookfield (Type RVT) viscometer at 25°C ± 1°C at 100 rpm using an appropriate spindle of the Brookfield RV-spindle set and is specified in mPa·s. Based on his technical knowledge, the skilled person will select a spindle from the Brookfield RV-spindle set which is suitable for the viscosity range to be measured. For example, for a viscosity range between 200 and 800 mPa·s the spindle number 3 may be used, for a viscosity range between 400 and 1 600 mPa·s the spindle number 4 may be used, and for a viscosity range between 800 and 3 200 mPa·s the spindle number 5 may be used.

### Residues

The residues were determined in accordance with ISO 787/7.

### Tappi-whiteness

Pigment whiteness R457 was measured using an ELREPHO 3000 from the company Datacolor according to ISO 2469:1994 (DIN 53145-2:2000 and DIN 53146:2000).

### Whiteness CIE L*, a*, b*

The CIELAB *L*^{∗}*, a*^{∗}, *b*^{∗} coordinates were measured using an ELREPHO 3000 from the company Datacolor according to DIN 6174 and barium sulphate as standard.

### R_{y} (C/2°)

*R_{y}* (C/2°) was determined in accordance with DIN 53163.

### 3. Examples

### Example 1: CaCO₃ slurry A with 75% solid content

Three samples of calcium carbonate slurry A were autoclaved. One autoclaved slurry was inoculated with a defined bacterial inoculum in order to receive a sample with 10³ cfu/mL and one with 10⁴ cfu/mL. The three different slurry samples (autoclaved, 10³ cfu/mL, 10⁴ cfu/mL) were measured 2-5 times with the Glomax^{®} 20/20 Luminometer (available from Promega) and the BacTiter Glo Reagent^{®} (available from Promega) and were plated in parallel on TSA plates in order to quantify the bacteria.

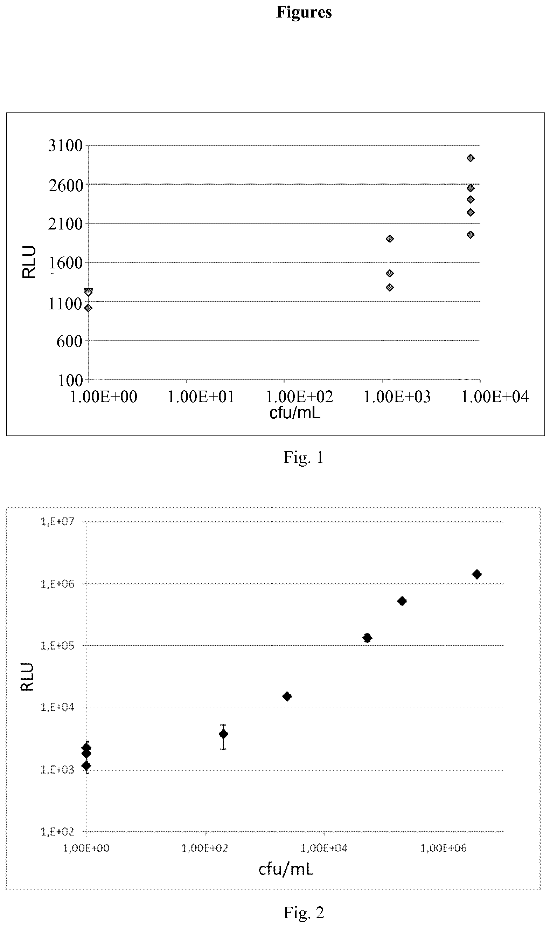

The results for the calcium carbonate slurries A with 75% solid content, tested without further dilution (overall dilution by addition of test reagents was 2) are shown in Fig. 1. Assay sensitivity was about 1E3 cfu/mL as defined by all measured samples above background level (here about 1 100 RLU). RLU was measured with Glomax^{®} 20/20 Luminometer (available from Promega) versus cfu/mL evaluated on TSA. Datapoints represent the mean of triplicates with standard deviation.

### Example 2: CaCO₃ slurry B 60 with 70% solid content

A sample of the contaminated calcium carbonate slurry B was autoclaved. For different points of the curve, a 10-fold serial dilution of the contaminated slurry in the autoclaved slurry was prepared as follows:
- 1:10 dilution: 1 mL contaminated slurry (original contaminated calcium carbonate slurry B) + 9mL autoclaved calcium carbonate slurry B
- 1:100 dilution: 1mL of the 1:10 diluted slurry + 9 mL autoclaved calcium carbonate slurry B
- 1:1 000 dilution: 1 mL of the 1:100 diluted slurry + 9 mL autoclaved calcium carbonate slurry B
- 1:10 000 dilution: 1 mL of the 1:1 000 diluted slurry + 9 mL autoclaved calcium carbonate slurry B

Then the different diluted slurries were tested with the Glomax^{®} 20/20 Luminometer (available from Promega; 3 measurements of each diluted slurry) and the BacTiter Glo Reagent^{®} (available from Promega) and were plated on TSA in 10-fold dilutions in order to quantify the bacteria on the TSA plates.

The results are as follows:
Autoclaved calcium carbonate slurry B for background level:
   o <100 cfu/mL on TSA, 1 862 RLU with Luminometer
Contaminated calcium carbonate slurry B:
   ∘ 200 cfu/mL on TSA, 3 729 RLU with Luminometer
   ∘ 2.36E+03 cfu/mL on TSA, 15 090 RLU with Luminometer
   ∘ 5.18E+04 cfu/mL on TSA, 133 948 RLU with Luminometer
   ∘ 1.98E+05 cfu/mL on TSA, 825 758 RLU with Luminometer
   ∘ 3.60E+06 cfu/mL on TSA, 1 417 575 RLU with Luminometer

The results for the CaCO₃ slurry calcium carbonate slurry B with 70% solid content, tested without further dilution (overall dilution by addition of test reagents was 2) are also shown in Fig. 2. Assay sensitivity was about 1E3 cfu/mL as defined by all measured samples above background level (here about 5E3 RLU). RLU was measured with Glomax^{®} 20/20 Luminometer (available from Promega) versus cfu/mL evaluated on TSA. Datapoints represent the mean of triplicates with standard deviation.

### Example 3: CaCO₃ slurry C with 50% solid content

A sample of the contaminated calcium carbonate slurry C was autoclaved. For different points of the curve, a 10-fold serial dilution of the contaminated slurry in the autoclaved slurry was prepared:
- 1:10 dilution: 1 mL contaminated slurry (original contaminated calcium carbonate slurry C) + 9 mL autoclaved calcium carbonate slurry C
- 1:100 dilution: 1 mL of the 1:10 diluted slurry + 9 mL autoclaved calcium carbonate slurry C
- 1: 1 000 dilution: 1 mL of the 1:100 diluted slurry + 9 mL autoclaved calcium carbonate slurry C

Then the different diluted slurry's were tested with the Glomax^{®} 20/20 Luminometer (available from Promega; 3 measurements of each diluted slurry) and the BacTiter Glo Reagent^{®} (available from Promega) and were plated on TSA in 10-fold dilutions in order to quantify the bacteria on the TSA plates.

The results are as follows:
Autoclaved calcium carbonate slurry C for background level:
   o <100 cfu/mL on TSA, 12 464 RLU
Contaminated calcium carbonate slurry C:
   ∘ 100 cfu/mL on TSA, 15 356 RLU with Luminometer
   ∘ 800 cfu/mL on TSA, 19 397 RLU with Luminometer
   o 5.36E+03 cfu/mL on TSA, 68 358 RLU with Luminometer
   o 5.25E+04 cfu/mL on TSA, 476 893 RLU with Luminometer

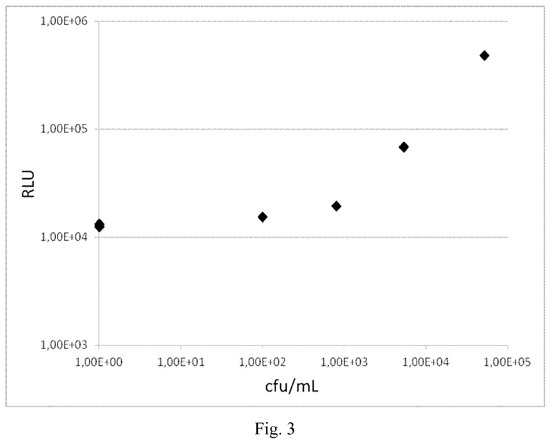

The results for the calcium carbonate slurry C with 50% solid content, tested without further dilution (overall dilution by addition of test reagents was 2) are also shown in Fig. 3. Assay sensitivity was about 1E2 cfu/mL as defined by all measured samples above background level (here about 20 000 RLU). RLU was measured with Glomax^{®} 20/20 Luminometer (available from Promega) versus cfu/mL evaluated on TSA. Datapoints represent the mean of triplicates with standard deviation.

## Claims

1. A method for detection of or determining an amount of microbial contamination in an aqueous preparation, the method comprising the steps of
a) providing an aqueous preparation comprising at least one particulate material,
b) providing at least one lytic agent,
c) contacting the aqueous preparation of step a) with the at least one lytic agent of step b) at a concentration and for a time sufficient to lyse a microbial contamination present in the aqueous preparation such that ATP which is set free from the microbial contamination is dissolved in the aqueous preparation, and
d) determining an amount of ATP content present in the aqueous preparation of step c), wherein the presence of microbial contamination is detected or the amount of microbial contamination is determined when the amount of ATP content is > 0,
wherein the aqueous preparation of step a) has a solids content from 10.0 to 82.0 wt.-%, based on the total weight of the aqueous preparation,
wherein the aqueous preparation of step a) is not diluted beyond a factor of 2 between method steps a) to d), and
wherein the aqueous preparation is an aqueous suspension.

2. The method according to claim 1, wherein the at least one particulate material is a white mineral having a whiteness of *R_{y}* of at least 65% measured according to DIN 53163.

3. The method according to any one of claims 1 or 2, wherein the at least one particulate material of step a) is selected from the group comprising natural ground calcium carbonate, natural and/or synthetic precipitated calcium carbonate, dolomite, surface-modified calcium carbonate, hydromagnesite, hydroxyapatite, kaolin, talcum, barite, aluminium hydroxide, aluminium silicate, titanium dioxide and mixtures thereof, and preferably the at least one particulate material comprises natural ground calcium carbonate and/or synthetic precipitated calcium carbonate.

4. The method according to any one of claims 1 to 3, wherein the aqueous preparation of step a) has
(i) a pH value of from 2 to 12, preferably from 6 to 12 and more preferably from 7 to 10.5, and/or
(ii) a solids content from 20.0 to 80.0 wt.-%, based on the total weight of the aqueous preparation.

5. The method according to any one of claims 1 to 4, wherein the aqueous preparation of step a) is a paper making formulation, a paper coating formulation, fibre formulation, food formulation, pharmaceutical formulation, cosmetic formulation, plastic formulation and/or a paint formulation.

6. The method according to any one of claims 1 to 5, wherein the at least one particulate material is not separated from the aqueous phase of the aqueous preparation between method steps a) to d).

7. The method according to any one of claims 1 to 6, wherein the ATP is originated from a microbial contamination comprising gram-negative bacteria, gram-positive bacteria, fungi, moulds, yeasts, algae or mixtures thereof.

8. The method according to any one of claims 1 to 7, wherein the at least one lytic agent of step b) is selected from the group comprising trichloroacetic acid (TCA), perchloric acid (PCA), citric acid, cetyl trimethylammonium bromide (CTAB), dodecyl trimethyl ammonium bromide (DTAB), chlorhexidine (CHEX), ultrasonication, other nonionic, anionic and cationic detergents, bacterial specific lytic enzymes such as lysostaphin or lysozyme, proteinase such as proteinase K, antibiotics, alkylglucoside or alkylthioglucoside, betane detergents, quaternary ammonium salts, protamines, amines, and cationic, antibacterial, pore forming, membrane-active, and/or cell wall-active polymers, bacteriophage, surfactants such as triton X, and mixtures thereof.

9. The method according to any one of claims 1 to 8, wherein the at least one lytic agent of step b) is provided together with a sufficient amount of at least one chelating agent, preferably EDTA.

10. The method according to any one of claims 1 to 9, wherein the amount of ATP content is determined by using a luciferin/luciferase reagent.

11. The method according to any one of claims 1 to 10, wherein steps c) and d) are carried out simultaneously, or separately in the given order.

12. The method according to any one of claims 1 to 11, wherein steps c) and d) are carried out simultaneously by using a luciferin/luciferase reagent containing at least one lytic agent.

13. The method according to any one of claims 11 to 12, wherein the presence of microbial contamination is detected or the amount of microbial contamination is determined in step d) by monitoring the luminescence signal.

14. The method according to any one of claims 1 to 13, wherein the presence of microbial contamination is detected or the amount of microbial contamination is determined in step d) by using a luminometer.

## Patentansprüche

1. Verfahren zum Erfassen oder Bestimmen einer Menge einer mikrobiellen Kontamination in einer wässrigen Zubereitung, wobei das Verfahren die folgenden Schritte umfasst
a) Bereitstellen von einer wässrigen Zubereitung, umfassend mindestens einen Feststoff,
b) Bereitstellen von mindestens einem lytischen Agens,
c) Kontaktieren der wässrigen Zubereitung von Schritt a) mit dem mindestens einen lytischen Agens von Schritt b) in einer Konzentration und für einen Zeitraum, die ausreichend sind, um eine in der wässrigen Zubereitung vorhandene mikrobielle Kontamination derart zu lysieren, sodass ATP, das von der mikrobiellen Kontamination freigesetzt wird, in der wässrigen Zubereitung aufgelöst wird, und
d) Bestimmen einer Menge von einem ATP-Gehalt, der in der wässrigen Zubereitung von Schritt c) vorhanden ist, wobei die Anwesenheit einer mikrobiellen Kontamination erfasst oder die Menge der mikrobiellen Kontamination bestimmt wird, wenn die Menge des ATP-Gehalts > 0 beträgt,
wobei die wässrige Zubereitung von Schritt a) einen Feststoffgehalt von 10,0 bis 82,0 Gew.-% basierend auf dem Gesamtgewicht der wässrigen Zubereitung aufweist,
wobei die wässrige Zubereitung von Schritt a) zwischen den Verfahrensschritten a) bis d) nicht über einen Faktor von 2 hinaus verdünnt wird und
wobei die wässrige Zubereitung eine wässrige Suspension ist.

2. Verfahren nach Anspruch 1, wobei der mindestens eine Feststoff ein weißes Mineral ist, das einen Weißheitsgrad nach R_{y} von mindestens 65 %, gemessen nach DIN 53163, aufweist.

3. Verfahren nach einem der Ansprüche 1 oder 2, wobei der mindestens eine Feststoff von Schritt a) ausgewählt ist aus der Gruppe umfassend natürliches gemahlenes Calciumcarbonat, natürliches und/oder synthetisches gefälltes Calciumcarbonat, Dolomit, oberflächenmodifiziertes Claciumcarbonat, Hydromagnesit, Hydroxyapatit, Kaolin, Talkum, Baryt, Aluminiumhydroxid, Aluminiumsilikat, Titandioxid und Gemische davon und wobei der mindestens eine Feststoff bevorzugt natürliches gemahlenes Calciumcarbonat und/oder synthetisches gefälltes Calciumcarbonate umfasst.

4. Verfahren nach einem der Ansprüche 1 bis 3, wobei die wässrige Zubereitung von Schritt a)
(i) einen pH-Wert von 2 bis 12, bevorzugt von 6 bis 12 und mehr, vorzugsweise von 7 bis 10,5 und/oder
(ii) einen Feststoffgehalt von 20,0 bis 80,0 Gew-% basierend auf dem Gesamtgewicht der wässrigen Zubereitung aufweist.

5. Verfahren nach einem der Ansprüche 1 bis 4, wobei die wässrige Zubereitung von Schritt a) eine Papierherstellungsformulierung, eine Papierbeschichtungsformulierung, Faserformulierung, Lebensmittelformulierung, pharmazeutische Formulierung, kosmetische Formulierung, Kunststoffformulierung und/oder eine Lackformulierung ist.

6. Verfahren nach einem der Ansprüche 1 bis 5, wobei der mindestens eine Feststoff zwischen den Verfahrensschritten a) bis d) nicht aus der wässrigen Phase der wässrigen Zubereitung abgeschieden wird.

7. Verfahren nach einem der Ansprüche 1 bis 6, wobei das ATP aus einer mikrobiellen Kontamination stammt, umfassend gramnegative Bakterien, grampositive Bakterien, Pilze, Schimmel, Hefepilze, Algen oder Gemische davon.

8. Verfahren nach einem der Ansprüche 1 bis 7, wobei das mindestens eine lytische Agens von Schritt b) ausgewählt ist aus der Gruppe umfassend Trichloressigsäure (TCA), Perchlorsäure (PCA), Zitronensäure, Cetyltrimethylammoniumbromid (CTAB), Dodecyltrimethylammoniumbromid (DTAB), Chlorhexidin (CHEX), Ultraschallbehandlung, andere nichtionische, anionische und kationische Detergenzien, bakterienspezifische Lysoenzyme, wie z. B. Lysostaphin oder Lysozyme, Proteinase, wie z. B. Proteinase K, Antibiotika, Alkylglucosid oder Alkylthioglucosid, Betaindetergenzien, quaternäre Ammoniumsalze, Protamine, Amine und kationische, antibakterielle, porenbildende, membranaktive und/oder wandaktive Polymere, Bakteriophage, Tenside, wie z. B. Triton X und Gemische davon.

9. Verfahren nach einem der Ansprüche 1 bis 8, wobei das mindestens eine lytische Agens von Schritt b) zusammen mit einer ausreichenden Menge von mindestens einem Chelatbildner, bevorzugt EDTA, bereitgestellt ist.

10. Verfahren nach einem der Ansprüche 1 bis 9, wobei die Menge des ATP-Gehalts unter Verwendung eines Luciferin-/Luciferasereagens bestimmt wird.

11. Verfahren nach einem der Ansprüche 1 bis 10, wobei die Schritte c) und d) gleichzeitig oder separat in einer bestimmten Reihenfolge durchgeführt werden.

12. Verfahren nach einem der Ansprüche 1 bis 11, wobei die Schritte c) und d) gleichzeitig unter Verwendung eines Luciferin-/Luciferasereagens, das mindestens ein lytisches Agens enthält, durchgeführt werden.

13. Verfahren nach einem der Ansprüche 11 bis 12, wobei in Schritt d) die Anwesenheit einer mikrobiellen Kontamination erfasst oder die Menge der mikrobiellen Kontamination bestimmt wird, indem das Lumineszenzsignal überwacht wird.

14. Verfahren nach einem der Ansprüche 1 bis 13, wobei in Schritt d) die Anwesenheit einer mikrobiellen Kontamination erfasst oder die Menge der mikrobiellen Kontamination bestimmt wird, indem ein Luminometer verwendet wird.

## Revendications

1. Procédé pour la détection ou la détermination d'un niveau de contamination microbienne dans une préparation aqueuse, le procédé comprenant les étapes consistant
a) à fournir une préparation aqueuse comprenant au moins un matériau particulaire,
b) à fournir au moins un agent lytique,
c) à mettre en contact la préparation aqueuse de l'étape a) avec le au moins un agent lytique de l'étape b) à une concentration et pendant un temps suffisants pour lyser une contamination microbienne présente dans la préparation aqueuse de telle sorte que l'ATP qui est libérée de la contamination microbienne, soit dissoute dans la préparation aqueuse, et
d) à déterminer une quantité de teneur en ATP présent dans la préparation aqueuse de l'étape c), dans lequel la présence d'une contamination microbienne est détectée ou le niveau de contamination microbienne est déterminé lorsque la quantité de teneur en ATP est > 0,
dans lequel la préparation aqueuse de l'étape a) présente une teneur en matières solides de 10,0 à 82,0 % en poids, sur la base du poids total de la préparation aqueuse,
dans lequel la préparation aqueuse de l'étape a) n'est pas diluée au-delà d'un facteur de 2 entre les étapes a) à d) du procédé et
dans lequel la préparation aqueuse est une suspension aqueuse.

2. Procédé selon la revendication 1, dans lequel le au moins un matériau particulaire est un minéral blanc présentant une blancheur de *R_{y}* d'au moins 65 % mesurée selon la norme DIN 53163.

3. Procédé selon l'une quelconque des revendications 1 à 2, dans lequel le au moins un matériau particulaire de l'étape a) est sélectionné dans le groupe comprenant du carbonate de calcium naturel broyé, du carbonate de calcium précipité naturel et/ou synthétique, de la dolomie, du carbonate de calcium modifié en surface, de l'hydromagnésite, de l'hydroxyapatite, du kaolin, du talc, de la barytine, de l'hydroxyde d'aluminium, du silicate d'aluminium, du dioxyde de titane et des mélanges de ceux-ci et, de préférence, le au moins un matériau particulaire comprend du carbonate de calcium naturel broyé et/ou du carbonate de calcium précipité synthétique.

4. Procédé selon l'une quelconque des revendications 1 à 3, dans lequel la préparation aqueuse de l'étape a) présente
(i) une valeur de pH de 2 à 12, de préférence de 6 à 12 et, plus préférentiellement, de 7 à 10,5 et/ou
(ii) une teneur en matières solides de 20,0 à 80,0 % en poids sur la base du poids total de la préparation aqueuse.

5. Procédé selon l'une quelconque des revendications 1 à 4, dans lequel la préparation aqueuse de l'étape a) est une formulation de fabrication de papier, une formulation de revêtement de papier, une formulation de fibre, une formulation alimentaire, une formulation pharmaceutique, une formulation cosmétique, une formulation plastique et/ou une formulation de peinture.

6. Procédé selon l'une quelconque des revendications 1 à 5, dans lequel le au moins un matériau particulaire n'est pas séparé de la phase aqueuse de la préparation aqueuse entre les étapes a) à d) du procédé.

7. Procédé selon l'une quelconque des revendications 1 à 6, dans lequel l'ATP provient d'une contamination microbienne comprenant des bactéries à Gram négatif, des bactéries à Gram positif, des champignons, des moisissures, des levures, des algues ou des mélanges de ceux-ci.

8. Procédé selon l'une quelconque des revendications 1 à 7, dans lequel le au moins un agent lytique de l'étape b) est sélectionné dans le groupe comprenant l'acide trichoroacétique (TCA), l'acide perchlorique (PCA), l'acide citrique, le brimure de cétyltriméthylammonium (CTAB), le bromure de dodécyltriméthylammonium (DTAB), la chlorhexidine (CHEX), une ultrasonication, d'autres détergents non ioniques, anioniques et cationiques, des enzymes lytiques spécifiques à la bactérie telles que la lysostaphine ou le lysozyme, une protéinase telle que la protéinase K, les antibiotiques, l'alkylglucoside ou l'alkylthioglucoside, des détergents à base de bétaïne, des sels d'ammonium quaternaire, des protamines, des amines et des polymères cationiques, antibactériens, formant des pores, à membrane active et/ou à paroi cellulaire active, un bactériophage, des tensioactifs tels que le triton X, et des mélanges de ceux-ci.

9. Procédé selon l'une quelconque des revendications 1 à 8, dans lequel le au moins un agent lytique de l'étape b) est fourni conjointement avec une quantité suffisante d'au moins un agent chélateur, de préférence l'EDTA.

10. Procédé selon l'une quelconque des revendications 1 à 9, dans lequel la quantité de teneur en ATP est déterminée en utilisant un réactif à base de luciférine/luciférase.

11. Procédé selon l'une quelconque des revendications 1 à 10, dans lequel les étapes c) et d) sont réalisées en même temps ou séparément dans l'ordre donné.

12. Procédé selon l'une quelconque des revendications 1 à 11, dans lequel les étapes c) et d) sont réalisée en même temps en utilisant un réactif à base de luciférine/luciférase contenant au moins un agent lytique.

13. Procédé selon l'une quelconque des revendications 11 à 12, dans lequel la présence d'une contamination microbienne est détectée ou le niveau de contamination microbienne est déterminé à l'étape d) en surveillant le signal de luminescence.

14. Procédé selon l'une quelconque des revendications 1 à 13, dans lequel la présence d'une contamination microbienne est détectée ou le niveau de contamination microbienne est déterminé à l'étape d) en utilisant un luminomètre.
